Europäisches Patentamt

European Patent Office

Office européen des brevets

(11) Publication number: **0 376 903**
**A2**

(12) **EUROPEAN PATENT APPLICATION**

(21) Application number: **89830566.9**

(51) Int. Cl.5: **A61N 1/30**

(22) Date of filing: **22.12.89**

(30) Priority: **29.12.88 IT 6816788**

(43) Date of publication of application:
**04.07.90 Bulletin 90/27**

(84) Designated Contracting States:
**AT BE CH DE ES FR GB GR IT LI LU NL SE**

(71) Applicant: **Benedicenti, Alberico**
**Via Corsica 9/3F**
**I-16128 Genova(IT)**

(72) Inventor: **Benedicenti, Alberico**
**Via Corsica 9/3F**
**I-16128 Genova(IT)**

(74) Representative: **Notaro, Giancarlo et al**
**c/o Jacobacci-Casetta & Perani S.p.A. Via**
**Alfieri, 17**
**I-10121 Torino(IT)**

(54) **A portable electronic ion-transfer device.**

(57) The portable electronic ion-transfer device is a piece of equipment which enables charged chemical compounds (with negative polarity) - for example cosmetics - to be transferred through the skin by means of a transfer method effected by electrical charges of an intensity such as to damage neither the body nor the skin; by virtue of the method used, the compound is distributed uniformly within the regions treated.

EP 0 376 903 A2

# A portable electronic ion-transfer device

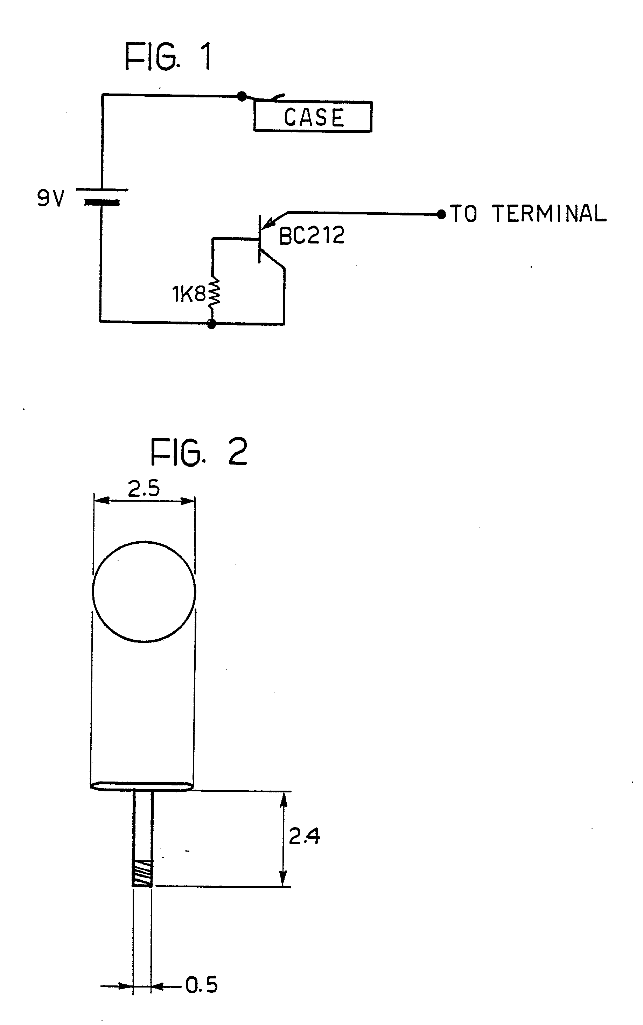

The transfer of charged chemical compounds is a subject which has already been confronted (iontophoresis); up to now, however, equipment which can transfer charged chemical compounds has used electrical energy taken from high-tension lines (220 V) with risks both to the operator and to the patients. The portable electronic ion-transfer device instead uses a new technique which limits the energy used which can thus be supplied by a simple 9V battery. As is seen from the diagram of Figure 1, the current delivered by the battery is regulated by a transistor which acts as a theoretical current-generator, thus enabling correct operation in any situation (e.g. almost flat batteries). The strength of the current (5-50 microAmperes) from the generator is limited with reference to an average value of the resistance of the human body of 350-500 KOhms (derived from average survey statistics) so that it can pass through the human body without damaging it. The contact surface of the terminal (Figure 2) has been designed to ensure that there are no point effects and its dimensions are calculated so as to be the optimum for providing a correct grip for a female hand (from statistics taken from the survey in question). Both surfaces are plated with 24 carat gold, thus ensuring that the charge is distributed uniformly and the contact resistance is reduced to a value of the order of tenths of an Ohm.

The apparatus is constituted by the following parts, shown in the appended drawings: a grip; a terminal; a top cover; a bottom cover and an electronic circuit.

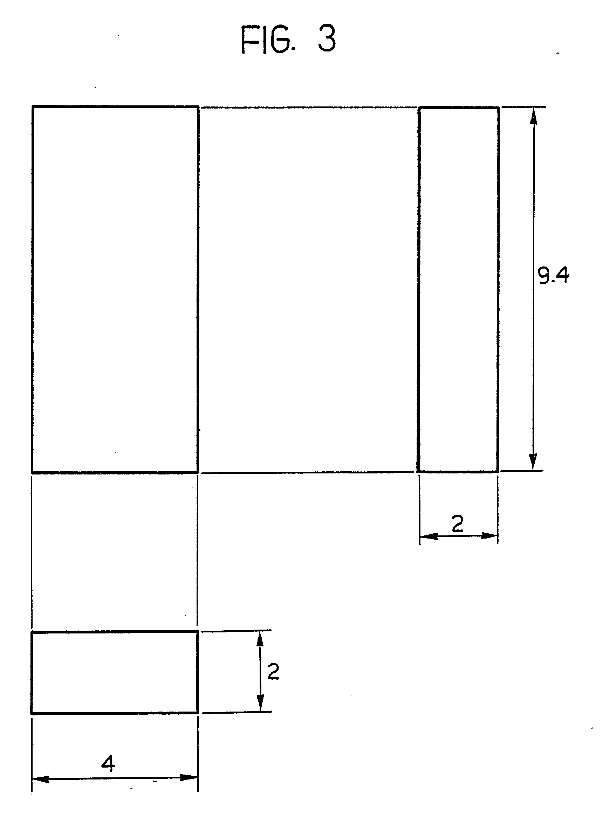

The grip is parallelepipedal, is plated with 24 carat gold and is shown in Figure 3.

The terminal is plated with 24 carat gold and is shown in Figure 2; the lower end is a standard-pitch screw which is screwed into the top cover.

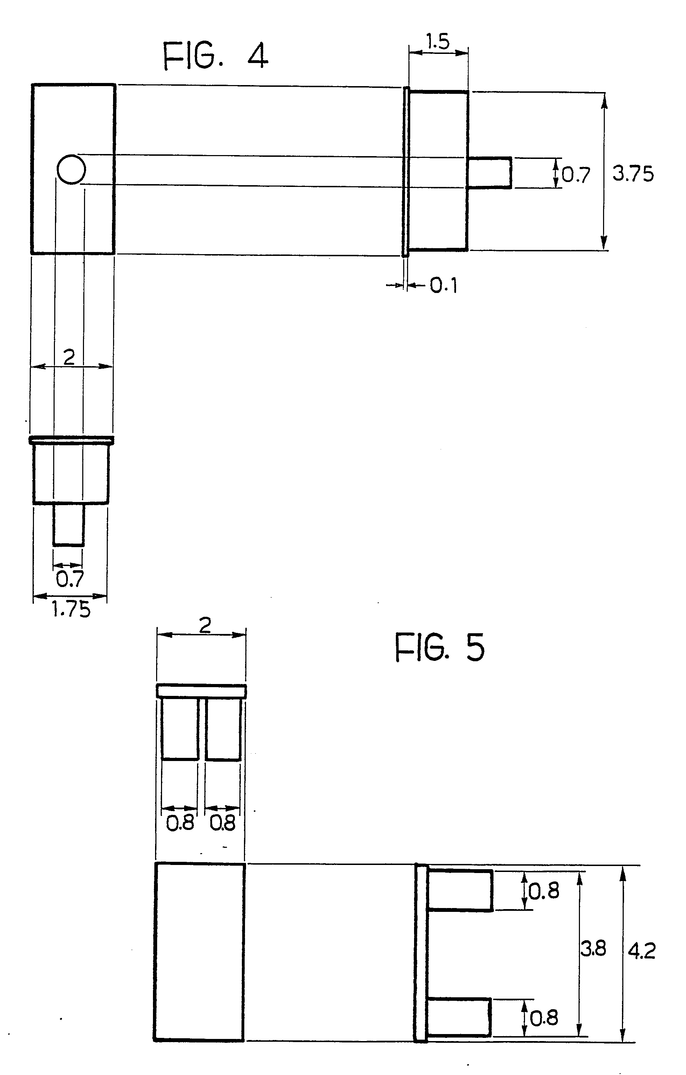

The top cover is shown in Figure 4; it is shaped so that, once assembled, it cannot be removed from the grip and thus provides a secure and firm support for the terminal.

The bottom cover shown in Figure 5, on the other hand, is removable for easy replacement of the battery.

The electronic circuit, the layout of which is shown in Figure 1, is formed physically on a 1 cm x 2 cm vetronit or bakelite plate.

The parts are assembled as follows:
one of the poles of the contact plate for the 9V battery is soldered to the electronic circuit, whilst its other pole is soldered to a contact tongue. The electronic circuit is then screwed directly on to the base of the top cover; the assembly is inserted in the grip, care being taken that the contact tongue is situated between the side edge of the top cover and the grip. The bottom cover is then inserted in the grip. Finally, the terminal is simply screwed into the top cover.

Naturally, the structural principle of the invention remaining the same, its details and forms of embodiment may be varied widely with respect to those described and illustrated purely by way of example, without thereby departing from the scope of the present invention.

## Claims

1. A method for transferring charged chemical compounds through the skin, characterised in that it makes use of a suitably prepared product by means of a galvanic generator which can generate a charge of known intensity.

2. A method according to Claim 1, characterised in that the energy source used is a 9 volt battery.

3. A method according to Claim 1 or Claim 2, characterised in that the contact with the human body is limited to two terminals.

4. A method according to any one of Claims 1 to 3, characterised in that the two terminals are gold-plated in order to minimise the contact resistance.

5. A method according to any one of the preceding claims, characterised in that the charge density on the transfer surface is that necessary and sufficient to activate a product designed so as to be suitable for ionic transfer.

# FIG. 1

CASE

9V

BC212

TO TERMINAL

1K8

# FIG. 2

2.5

2.4

0.5

# FIG. 3

# FIG. 4

# FIG. 5